# EUROPEAN PATENT SPECIFICATION

(11) **EP 4 126 131 B1**
(45) Date of publication and mention of the grant of the patent: **17.12.2025**
(21) Application number: 21715627.2
(22) Date of filing: 31.03.2021
(51) Int. Cl.: A61M 5/32, A61M 5/42, A61M 5/158, A61M 25/02

(54) **PATCH FOR USE WITH AN INJECTOR DEVICE**
PFLASTER ZUR VERWENDUNG MIT EINER INJEKTIONSVORRICHTUNG
PATCH À UTILISER AVEC UN DISPOSITIF D'INJECTION

(30) Priority: 03.04.2020 EP 20315111
(43) Date of publication of application: 08.02.2023
(73) Proprietor: SANOFI, 75008 Paris (FR)
(72) Inventor: DASBACH, Uwe, 65926 Frankfurt am Main (DE); SCHEINERT, Kai, 65926 Frankfurt am Main (DE); AUERNHAMMER, Daniel, 65926 Frankfurt am Main (DE); SCHAUDERNA, Florian, 65926 Frankfurt am Main (DE); KEMP, Thomas Mark, Melbourn Herts Cambridgeshire SG8 6DP (GB); SCHULLER, Tim, Melbourn Herts Cambridgeshire SG8 6DP (GB); WILSON, Robbie, Melbourn Herts Cambridgeshire SG8 6DP (GB); NOBLE, Michael, Melbourn Herts Cambridgeshire SG8 6DP (GB); MCGINLEY, Ryan Anthony, Melbourn Herts Cambridgeshire SG8 6DP (GB)
(74) Representative: Brown, Alexander Edward
(86) International application number: PCT/EP2021/058493
(87) International publication number: WO 2021/198364

(56) References cited:
- CN-A- 105 944 227
- CN-A- 109 498 272
- US-A- 4 988 341
- US-A1- 2014 039 451
- US-B1- 10 195 366

## Description

### FIELD OF INVENTION

The present invention relates to a patch for placement on skin and for use with an injector device for a medicament.

### BACKGROUND

Injector devices, for example auto-injectors, typically have a sealed container of medicament, and a needle for injection of the medicament into a patient. A plunger or piston within the medicament container can be moved into the medicament container to dispense medicament through the needle for injection to a patient.

Some patients may experience discomfort when an injection needle is inserted into the skin at the injection site or during delivery of the medicament. The level of discomfort may vary depending on numerous factors, some relating to the medicament such as the temperature, volume and viscosity of the medicament, some relating to the injector device, such as size and insertion depth of the injection needle, or injection delivery time. Some patients may also feel uncomfortable with the anticipation of the injection process and the feel and sight of an injection needle before and during insertion into the skin.

CN 105 944 227 discloses a local analgesia patch comprising an adhesive layer and an elastic layer, wherein the elastic layer covers the upper surface of the adhesive layer. Two ends of the elastic layer are connected to two ends of the adhesive layer in a sealed mode; the middle of the elastic layer is separated from the adhesive layer, so that a cavity is formed; the cavity is of an arc-shaped structure which is provided with a needle therein; the needle is perpendicular to the adhesive layer; the needle is connected to a pressing head; the pressing head is arranged above the needle and is fixedly arranged on the upper surface of the elastic layer; and the cavity is filled with a liquid analgesic drug.

US 10 195 366 discloses an injection analgesia system to numb a patient's skin around an injection site. An injection window guides a caregiver's injection placement to a relatively small area while allowing standard injection procedures to be followed, such as stretching the skin and puncturing the site with a jabbing motion. The injection analgesia system has a needle shield and an analgesia, which are layered together and applied to a skin surface. A needle shield window and a analgesia window align to form the injection window.

US 4 988 341 discloses a sterilizing dressing which comprises a pair of cover sheets and a patch which is sandwiched between the two cover sheets. The patch is transparent and effective to reseal after being punctured by a needle. The patch includes a sterilizing agent. In use, the needle penetrates the patch in the dressing prior to penetrating the skin.

US2014/039451 discloses wearable injection guides which include: a rigid material formed to substantially conform in shape to a topography of a body region of an individual, the rigid material substantially impenetrable to an injection needle, and the rigid material including one or more injection needle access regions arranged in a treatment pattern.

CN 109 498 272 discloses an infusion bandage comprising a sterilization anti-inflammation block, an elastic pressing block, first release paper and a first bonding sheet. By the aid of the first bonding sheet, the sterilization anti-inflammation block can be fixed at a needle hole of a patient when infusion is implemented and after infusion is completed, so that the sterilization anti-inflammation block covers and disinfects the needle hole.

### SUMMARY

According to the present disclosure, there is provided a patch for placement on the skin and for use with an injector device, the patch comprising a first layer on a first side of the patch, a second layer on a second side of the patch opposite to the first side, a sealed fluid reservoir between the first and second layers, a fluid contained within the fluid reservoir, and a moveable rupturing element configured to pierce and rupture the fluid reservoir to allow release of the fluid out of the fluid reservoir. The patch may also comrpise an aperture extending at least partially through the patch to receive an injection needle of an injector device.

The fluid reservoir may be defined by the first and second layers. The first and second layers may be separate elements bonded, welded, or otherwise connected together, or the first and second layers may be integrally formed.

The rupturing element may be moveable between an initial position and an activated position in which the rupturing element ruptures the fluid reservoir.

A biasing arrangement may be configured to bias the rupturing element into the initial position. This may advantageously help towards preventing unintended rupturing of the fluid reservoir.

The rupturing element may be disposed within the fluid reservoir. This may advantageously help towards providing a simpler patch to use, and/or avoid unintended contact with the components of the patch. Alternatively, the rupturing element may be disposed outside the fluid reservoir. The rupturing element may be a separate component to the first and second layers. This may advantageously help towards ease and cost reduction of manufacture.

The patch may comprise a support member. The rupturing element may be moveably mounted to the support member. This may advantageously help towards controlling relative movement of the rupturing element and support member during use. This may advantageously also help towards supporting an injection device in use with the patch.

The rupturing element may be integrally formed with the one of the first and second layers. This may advantageously help towards ease and reduced cost of manufacture.

The injection aperture may extend partially through the patch, and may extend through only one of the first and/or second layers of the patch. Alternatively, the injection apeture may extend entirely through the patch, such as through both the first and second layers of the patch. The injection aperture may be configured to receive an injection needle of an injector device, and/or to receive a portion of a body or housing of an injector device. The injection aperture may comprise an abutment or stepped portion such that part of an injector device may be reiceved in the injection aperture until the injector device reaches and contacts the abutment or stepped portion. This may advantageously help towards guiding use of an injection device with the patch.

At least one of the first and second layers may comprises a piercable membrane which can be pierced by the rupturing element to rupture the fluid reservoir.

The piercable membrane may include one or more regions of weakness at which the piercable membrane is more easily ruptured than at other regions of the piercable membrane. This may advantageously help towards ensuring effective rupturing of the reservoir in use.

The at least one region of weakness may comprise a region of reduced thickness in the piercable membrane.

The support element and rupturing element may surround the injection aperture. The injection aperture may be formed in one or both of the first and second layers. The injection aperture may be formed through the piercable membrane. This may advantageously help to ensure an injection needle of the injection device enters the skin. Alternatively, the piercable membrane may extend across the injection aperture. In such an embodiment, an injection needle of an injector device may pierce the piercable membrane during use of the patch. This may advantageously help towards ease of reduced cost of manufacture.

The injection aperture may extend at least partially through the patch and may comprise an axis. The axis may be defined centrally within the injection aperture. The injection aperture may be disposed substantially centrally on the patch. The support member and rupturing element may be moveable relative to each other in a direction of the injection aperture axis. This may advantageously facilitate movement of the injector device effecting relative movement of the support member and rupturing element.

A portion of the patch may comprise a skin-contacting surface, and the patch may further comprise a protective material layer removably adhered over the skin-contacting surface and which is intended to be removed before use of the patch. This may advantageously help protect the patch from external damage before use, and/or prevent contamination of the patch before use.

A portion of the patch may comprise a skin-contacting surface, and the skin-contacting surface may comprise an adhesive layer to secure the patch to the skin of a patient. This may advantageously help to secure the patch on the skin before and during use.

The patch may include a locking mechanism associated with the rupturing element and configured such that in a locked condition of the locking mechanism, the rupturing element is prevented from being moveable to rupture the fluid reservoir, and in an unlocked condition of the locking mechanism, to rupturing element is moveable to be able to rupture the fluid reservoir. This may advantageously help towards preventing unintended activation of the patch.

The locking mechanism may include a release element which is engagable by an injector device, and operable to move the locking mechanism into the unlocked condition when an injector device is pressed against the release element. This may advantageously help towards ensuring the patch is only activated once an injection device is in contact and/or engaged with the patch.

The patch may include a stop associated with the rupturing element and configured to restrict movement of the rupturing element beyond a predetermined range of movement. This may advantageously help towards preventing the rupturing element contacting or pressing onto the skin more than an intended degree during use, and to prevent or minimise discomfort in use.

The rupturing element may comprise a circular or substantially circular component, such as a circular or elliptical component. The rupturing element may surround a portion of the support member. This may advantageously help towards ease of manufacture and reliability of operation in use, and/or assist in guiding relative movement of the rupturing element and support member during use.

The rupturing element may comprise one or more pointed protrusions configured to pierce a component which defines the fluid reservoir in order to rupture the fluid reservoir.

The support member may comprise a substantially planar component, and/or a substantially planar portion, such as a plate or flange portion. The substantially planar portion may extend substantially parallel to one or both of the first and second layers, such as the membrane. This may advantageously help to support the respective layer(s).

The patch may comprise one or more fluid passages configured to allow fluid from the reservoir to pass towards the skin, and advantageously configured to allow fluid to flow into contact with the skin.

The biasing arrangement may comprise one or more biasing members. The biasing member(s) may be provided on the support member and/or on the rupturing element. The biasing member(s) may be formed integrally with the support member and/or rupturing element, or may be separate component(s) connected to the support member and/or rupturing element, such as by bonding, welding or other means of mechanical fastening. The biasing member(s) may comprise a resilient component and may comprise spring arm(s) and/or coil spring(s).

The fluid in the fluid reservoir may comprise a liquid. The fluid in the fluid reservoir may comprise one of an antiseptic, analgesic, anaesthetic, or sterilising agent. The fluid in the fluid reservoir may comprise a liquid which does not include a chemically or pharmacologically active substance.

The patch may include a temperature varying arrangement configured to be activated in use of the patch to apply a heating or cooling sensation to the skin of a patient.

The patch may further comprise a thermochromic element configured to display a plurality of colours dependent on a temperature of the patch.

Also provided is a system comprising a patch as described above, and an injector device for use with the patch, wherein the injector device comprises a housing configured to receive a container of medicament.

The injector device may comprise a container of medicament received within the housing.

Also provided is a method of use of a patch for placement on the skin and for use with an injector device, the patch comprising a first layer on a first side of the patch, a second layer on a second side of the patch opposite to the first side, a sealed fluid reservoir between the first and second layers, a fluid contained within the fluid reservoir, the method comprising moving a moveable rupturing element to rupture the fluid reservoir to allow release of the fluid out of the fluid reservoir.

The method may comprise placement of the patch on the skin. The method may comprise engaging an injector device with the patch, and movement of the moveable rupturing element by means of the injector device engaging with the patch. The method may comprise applying pressure on the patch via the injector device to move the moveable rupturing element.

The method may comprise moving the rupturing element between an initial position and an activated position in which the rupturing element ruptures the fluid reservoir.

The method may comprise the support member and rupturing element moving relative to each other, and such movement may be in a direction of the injection aperture axis.

The method may comprise removing a protective material layer adhered over the skin-contacting surface before use of the patch.

The method may comprise securing the patch to the skin with an adhesive layer on a skin contacting surface of the patch.

The method may comprise moving a locking mechanism associated with the rupturing element from a locked condition, in which the rupturing element is prevented from being moveable to rupture the fluid reservoir, to an unlocked condition of the locking mechanism, in which the rupturing element is moveable to be able to rupture the fluid reservoir.

The method may comprise engaging a release element by an injector device, to move the locking mechanism into the unlocked condition when an injector device is pressed against the release element.

These and other aspects of the invention will be apparent from and elucidated with reference to the embodiments described hereinafter.

### BRIEF DESCRIPTION OF THE DRAWINGS

Embodiments will now be described, by way of example only, with reference to the accompanying drawings, in which:
FIG. 1A is a schematic side view of an injector device including a removable cap;
FIG. 1B is a schematic side view of the injector device of FIG. 1A, with the cap removed from the housing;
FIG. 2 is a perspective view of an injection patch of a first embodiment;
FIG. 3 is a side view of the injection patch of FIG. 2 in place on a patient's skin and showing an injector device for use with the injection patch;
FIG. 4 is a cross-sectional view of the injection patch of FIG. 2 in an initial position;
FIG. 5 is a cross-sectional view of the injection patch of FIG. 2 in an activated position;
FIG. 6 is an exploded perspective view of the injection patch of FIG. 2 showing the component parts thereof;
FIG. 7 is a cross-sectional view of an injection patch of a second embodiment in an initial position;
FIG. 8 is a cross-sectional view of the injection patch of Figure 7 in an activated position;
FIG. 9 is an exploded perspective view of the injection patch of FIGS. 7 and 8 showing the component parts thereof;
FIG. 10 is a cross-sectional view of an injection patch of a third embodiment in an initial position;
FIG. 11 is a cross-sectional view of the injection patch of FIG. 10 in an activated position;
FIG 12 is an enlarged partial cross-sectional view of an injection patch of a fourth embodiment in a locked condition; and
FIG. 13 is an enlarged partial cross-sectional view of the injection patch of FIG. 12 in an unlocked condition.

### DETAILED DESCRIPTION

A drug delivery device, as described herein, may be configured to inject a medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 3 ml. Another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 50 ml). Yet another device may comprise a pre-filled syringe within a housing of the device. The syringe may be fixed within the housing or may be moveable within the housing, for example from a retracted position to an operation extended position.

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 17 and 29 Gauge.

The delivery devices described herein can also include one or more automated functions. For example, one or more of combining the needle and cartridge, needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may each be activated via an activation mechanism. Such an activation mechanism can include an actuator, for example, one or more of a button, a lever, a needle sleeve, or other activation component. Activation of an automated function may be a one-step or multi-step process. That is, a user may need to activate one or more activation components in order to cause the automated function. For example, in a one-step process, a user may depress a needle sleeve against their body in order to cause injection of a medicament. Other devices may require a multi-step activation of an automated function. For example, a user may be required to depress a button and retract a needle shield in order to cause injection.

In addition, activation of one automated function may activate one or more subsequent automated functions, thereby forming an activation sequence. For example, activation of a first automated function may activate at least two of combining the needle and cartridge, needle insertion, medicament injection, and needle retraction. Some devices may also require a specific sequence of steps to cause the one or more automated functions to occur. Other devices may operate with a sequence of independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

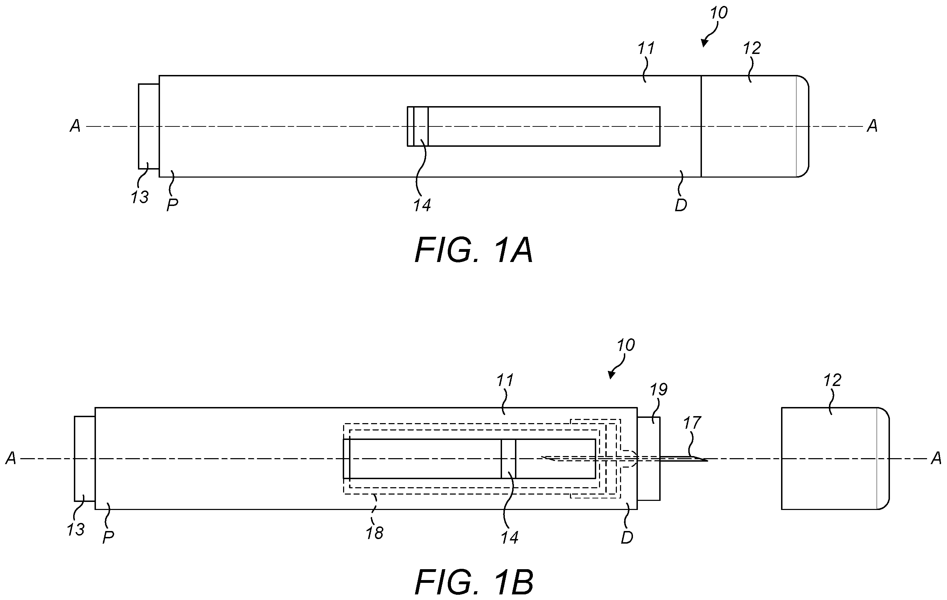

An exemplary drug delivery device 10 is shown in FIGS. 1A and 1B. Device 10, as described above, is configured to inject a medicament into a patient's body. Device 10 includes a housing 11 which typically contains a cartridge or pre-filled syringe that defines a reservoir containing the medicament to be injected, and the components required to facilitate one or more steps of the delivery process.

The device 10 can also include a cap 12 that can be detachably mounted to the housing 11. Typically, a user must remove cap 12 from housing 11 before device 10 can be operated.

As shown, housing 11 is substantially cylindrical and has a substantially constant diameter along the longitudinal axis A-A. The housing 11 has a distal region D and a proximal region P. The term "distal" refers to a location that is relatively closer to a site of injection, and the term "proximal" refers to a location that is relatively further away from the injection site.

Device 10 can also include a needle sleeve 19 coupled to housing 11 to permit movement of sleeve 19 relative to housing 11. For example, sleeve 19 can move in a longitudinal direction parallel to longitudinal axis A-A. Specifically, movement of sleeve 19 in a proximal direction can permit a needle 17 to extend from distal region D of housing 11.

Insertion of needle 17 can occur via several mechanisms. For example, needle 17 may be fixedly located relative to housing 11 and initially be located within an extended needle sleeve 19. Proximal movement of sleeve 19 by placing a distal end of sleeve 19 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 19.

Another form of insertion is "automated", whereby needle 17 moves relative to housing 11. Such insertion can be triggered by movement of sleeve 19 or by another form of activation, such as, for example, a button 13. As shown in FIGS. 1A and 1B, button 13 is located at a proximal end of housing 11. However, in other embodiments, button 13 could be located on a side of housing 11.

Other manual or automated features can include drug injection or needle retraction, or both. Injection is the process by which a bung or piston 14 is moved from a proximal location to a more distal location within the reservoir of the cartridge 18 in order to force a medicament from the cartridge 18 through needle 17. In some embodiments, a drive spring (not shown) is under compression before device 10 is activated. A proximal end of the drive spring can be fixed within proximal region P of housing 11, and a distal end of the drive spring can be configured to apply a compressive force to a proximal surface of piston 14. Following activation, at least part of the energy stored in the drive spring can be applied to the proximal surface of piston 14. This compressive force can act on piston 14 to move it in a distal direction. Such distal movement acts to compress the liquid medicament within the cartridge 18, forcing it out of needle 17.

Following injection, needle 17 can be retracted within sleeve 19 or housing 11. Retraction can occur when sleeve 19 moves distally as a user removes device 10 from a patient's body. This can occur as needle 17 remains fixedly located relative to housing 11. Once a distal end of sleeve 19 has moved past a distal end of needle 17, and needle 17 is covered, sleeve 19 can be locked. Such locking can include locking any proximal movement of sleeve 19 relative to housing 11.

Another form of needle retraction can occur if needle 17 is moved relative to housing 11. Such movement can occur if the cartridge 18 within housing 11 is moved in a proximal direction relative to housing 11. This proximal movement can be achieved by using a retraction spring (not shown), located in distal region D. A compressed retraction spring, when activated, can supply sufficient force to the cartridge 18 to move it in a proximal direction. Following sufficient retraction, any relative movement between needle 17 and housing 11 can be locked with a locking mechanism. In addition, button 13 or other components of device 10 can be locked as required.

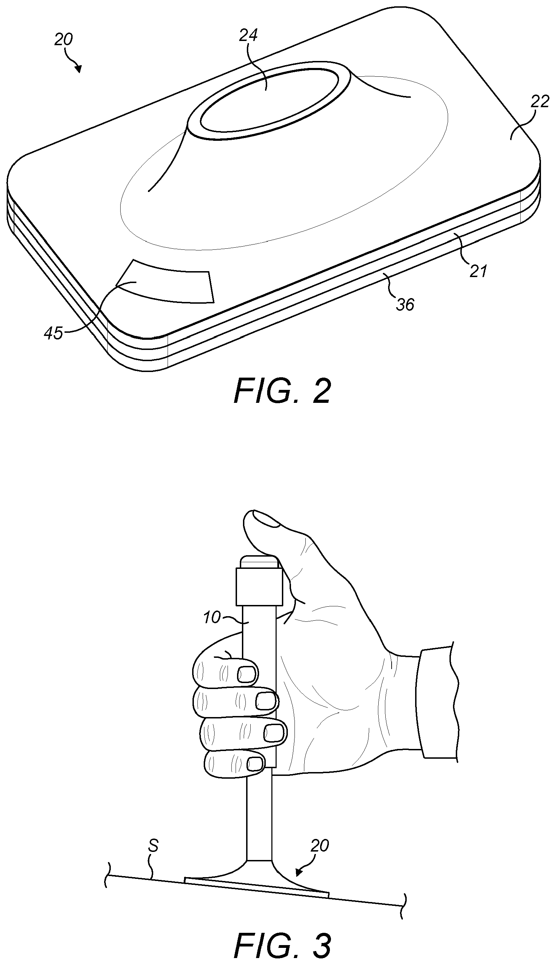

FIGS. 2 to 6 show an injection patch 20 of a first embodiment, suitable for use with an injector device, such as the injector device 10 shown in FIGS 1A and 1B. The injection patch 20 comprises a first layer 21 and a second layer 22. The first layer is disposed on a first side of the patch 20 which, in use, is intended to lie in contact with, or at least proximate, the skin S of a patient. The second layer 22 is disposed on an opposite side of the patch 20 from the first layer 21, and is on a side of the patch 20 that, in use, faces away from the skin of a user. In the exemplary embodiment shown, the first layer 21 comprises a membrane 21 and the second layer 22 comprises an open cup-shaped housing 22 defining an interior space. The membrane 21 is attached and sealed across an opening of the housing 22 to seal an enclosed interior space 23 between the first and second layers of the membrane 21 and the housing 22. The inner space 23 comprises a fluid reservoir containing a fluid. That is, the sealed fluid reservoir 23 is defined between the first and second layers 21, 22. In the first exemplary embodiment, the fluid reservoir is defined by the first and second layers 21, 22, although in alternative embodiments envisaged within the scope of the present disclosure, the reservoir 23 may be defined by other layers or components of the patch 20. Such alternative embodiments may comprise the reservoir disposed between the first and second layers 21, 22.

The fluid within the reservoir 23 of the exemplary embodiment may comprise a sterilising agent, for example alcohol or other liquid with sterilising properties. The fluid may additionally or alternatively comprise an analgesic or anaesthetic to reduce the sensation of an injection by an injection needle of an injector device 10 during use of the patch 20 (described in more detail hereafter). The fluid is advantageously a liquid. The liquid may comprise the substances and/or properties mentioned above. The liquid may alternatively comprise an inert substance, or a substance that does not have chemical, biological or pharmacologically active properties. In one embodiment, the liquid may comprise water.

The patch 20 includes in injection aperture 24 extending through the patch 20. The injection aperture 24 is configured for an injection needle 17 of an injector device 10 to extend through the injection aperture 24 during an injection process. The injection aperture 24 is provided by a cylindrical wall 25 portion of the housing 22 and a correspondingly shaped hole 26 in the membrane 21. The membrane 21 is sealed to an end surface of the cylindrical wall 25 around the hole 26.

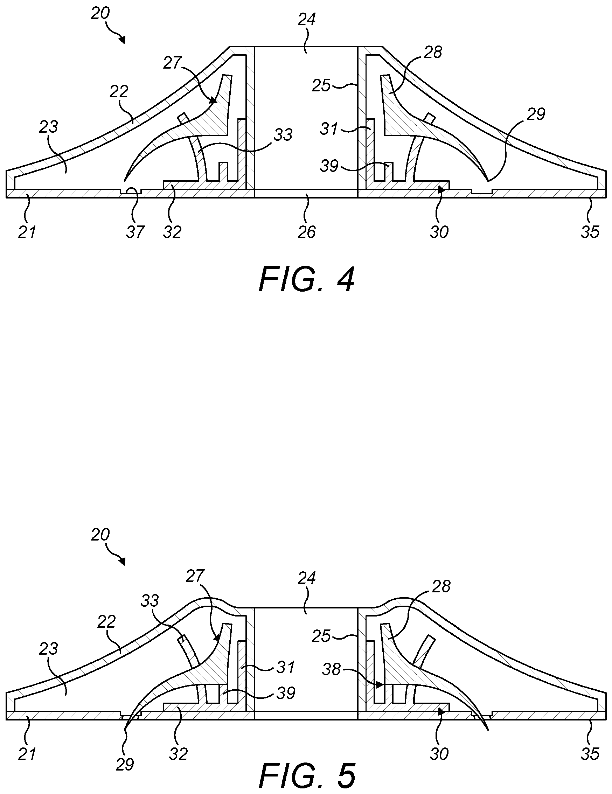

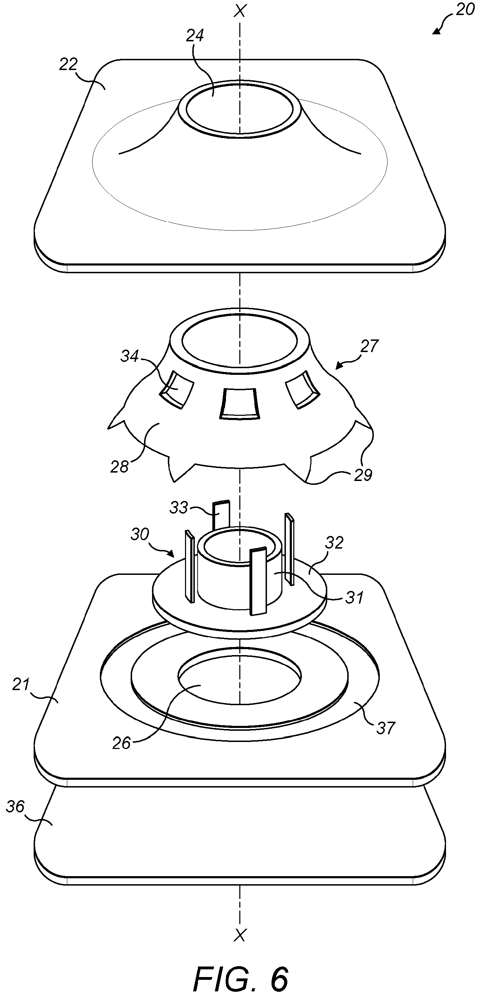

The patch 20 includes a moveable rupturing element 27 disposed within the fluid reservoir 23. The rupturing element 27 comprises a ring shaped body 28 with a plurality of pointed protrusions 29. The protrusions 29 are directed towards the membrane 21. The patch 20 further comprises a support member 30 about which the rupturing element 27 is moveably mounted. The support member 30 comprises a hollow cylindrical portion 31 and a flange 32 extending radially from one end of the hollow cylindrical portion 31 proximate the membrane 21. The rupturing element 27 is disposed around the hollow cylindrical portion 31 of the support member 30 and is moveable relative to the support member 30 about their common axis X-X (see Figure 6) from an initial position, shown in Figure 4, to an activated position, shown in Figure 5.

A biasing arrangement 33 is provided and configured to bias the rupturing element 27 into the initial position. In the exemplary embodiment shown, the biasing arrangement 33 comprises a biasing element 33 in the form of a plurality of resilient arms 33. These extend through slots 34 in the rupturing element 27. The slots 34 are configured such that as the rupturing element 27 moves from the initial position to the activated position, the resilient arms 33 are deflected and exert a force on the rupturing element 27 biasing it in a direction towards the initial position.

The cylindrical wall 25 of the housing 22 is disposed within the hollow cylindrical portion 31 of the support member 30. The membrane 21 is sealed around a peripheral edge of the housing 22 and around the end surface of the hollow cylindrical portion 31 as described above, such that the support member 30 and rupturing element 27 are sealed within the fluid reservoir 23.

In the exemplary embodiment, the membrane 21 comprises a skin-contacting surface of the patch 20. An adhesive 35 is provided on an outwardly-facing surface of the membrane 21 to enable the patch 20 to be adhered to the skin S of a patient. A protective material layer 36 is provided on the membrane 21. The protective material layer 36 is removable from the membrane 21 before use of the patch 20, but serves to protect the membrane 21 from contact, contamination or damage prior to use, for example during manufacture, transport and storage of the patch 20. Advantageously, the protective material layer 36 is of a greater thickness than the membrane 21. The protective material layer 36 may also be made of a tougher or more structurally resilient material than that of the membrane 21.

The membrane 21 includes a weakened region 37. The weakened region 37 is located in the location of the protrusions 29 of the rupturing element 27 when the rupturing element 27 is in the activated position. The weakened region 37 is shown as a single circular region, but may alternatively comprise a plurality of discrete regions or any suitable shape or size. The weakened region 37 may be formed integrally with the membrane 21, and may comprise a region of reduced thickness of the material of the membrane 21, or a region demarcated by one or more lines of weakening in the material of the membrane 21, such as score lines or lines of reduced material thickness. Alternatively, the weakened region 37 may comprise an aperture in the membrane 21 and a portion of a different rupturable material bonded over the aperture.

The support member 30 and rupturing element 27 are provided with a stop arrangement 38 which defines the position of the rupturing element 27 relative to the support member 30 in the activated position and prevents further movement of the rupturing element 27 relative to the support member 30 beyond the activated position. In the exemplary embodiment shown, the stop arrangement 38 comprises a plurality of posts 39 extending from the flange 32. In the activated position of the rupturing element 27, the rupturing element 27 abuts the posts 39, thereby preventing further movement of the rupturing element 27 beyond the activated position.

Use of the patch 20 of the first embodiment will now be described. A user first removes the protective material layer 36 exposing the adhesive layer 35. The user then places the patch 20 on the patient's skin at the intended injection site, with the location of the intended injection in the middle of the hole 26 in the membrane 21 and aligned with the injection aperture 24. (Herein, a user may comprise the patient in the context of a self-administered medicament, or may comprise a health care professional in the case of a health care professional administering medicament to a patient). The adhesive layer 35 helps retain the patch 20 in position on the patient's skin.

The user then prepares the injector device 10 for administering an injection, and places the distal end of the injector device 10 on the patch 20 with the axis A-A of the injector device 10 aligned with the axis X-X of the rupturing element 27 and hollow cylindrical portion 31 of the support member 30. The user then presses the injector device 10 down onto the patch 20, which causes an upper portion of the housing 22 to deform towards the skin of the patient and the rupturing element 27 to move from the initial position shown in Figure 4, to the activated position shown in Figure 5. The pointed protrusions 29 of the rupturing element 27 pierce the membrane 21. The piercing is facilitated by the protrusions 29 being located facing the weakened regions 37 of the membrane 21 such that those regions of the membrane 21 are relatively easily pierced.

The pierced membrane 21 allows the fluid from within the fluid reservoir 23 to be released onto the patient's skin at and around the injection site. Depending on the formulation of the liquid within the fluid reservoir, the released fluid may help to sterilised the injection site, and/or reduce the patient's sensation of touch or pain at the injection site. Thereby, subsequent progression of the injection process, in which the injection needle 17 extends through the injection aperture 24 and into the patient's skin S, is ensured to be in a sterile region of skin, and/or causes the patient a reduced discomfort as the needle 17 enters the skin and the medicament injection process progresses. The sensation of fluid from the ruptured reservoir 23 on the skin may provide a sensory distraction from the injection sensation, without active anaesthetic action. For example, if the fluid in the reservoir is water. The patch 20, and thereby the fluid within the reservoir, may be heated or cooled prior to use to provide a warming or cooling sensation on the skin in use, helping distraction from injection sensation. The fluid within the reservoir may alternatively comprise a pressurised gas such that sensation of gas release upon rupturing of the reservoir provides a distracting sensation. The quick pressure drop may also provide a cooling sensation. The fluid may yet further comprise a liquid that evaporates upon release from the reservoir 23, again further providing a cooling sensation to the skin at the injection site.

The posts 39 of the stop arrangement 38 abut the rupturing element 27 in the activated position and prevented the rupturing element 27 from moving beyond the activation position. The stop arrangement 38 thereby serves to prevent the protrusions 29 of the rupturing element 27 extending further through the membrane 21 which may otherwise cause them to press more firmly onto the patient's skin. This thereby helps towards preventing discomfort for the patient being caused by an unwanted degree of contact with the protrusions 29.

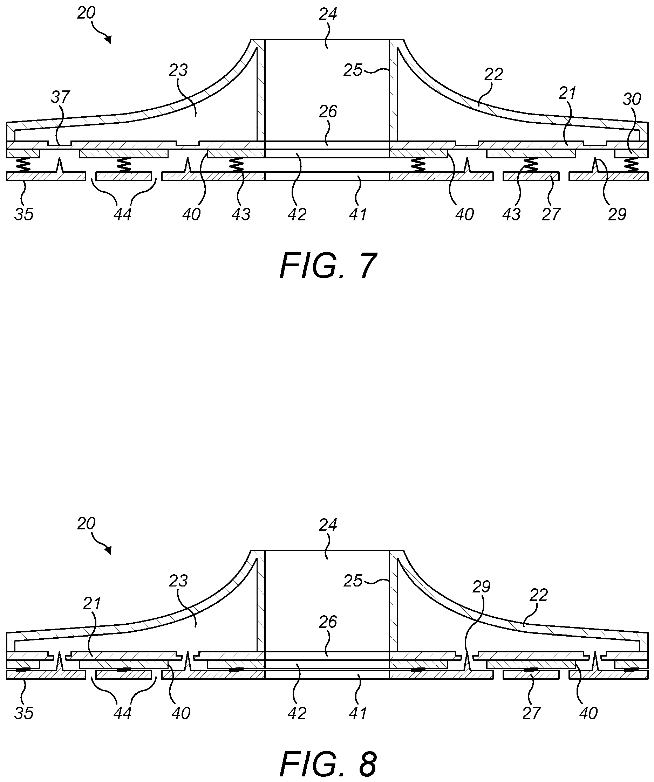

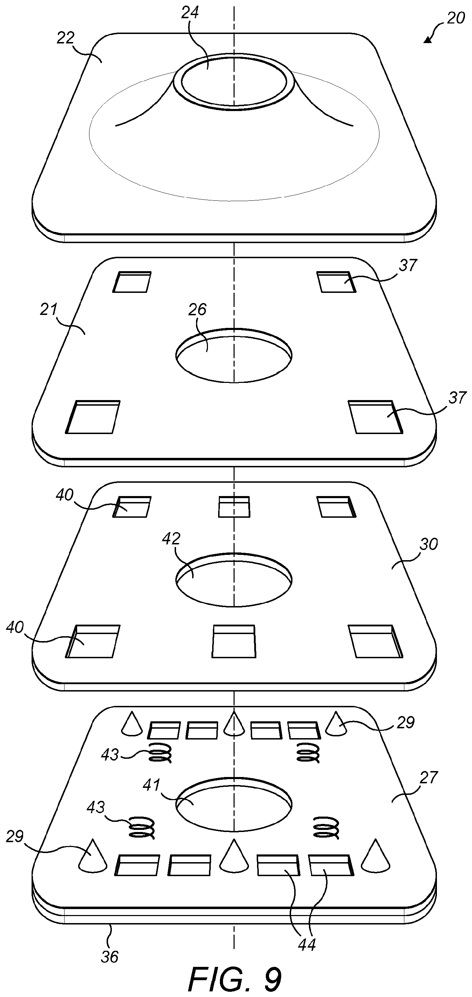

A patch 20 of a second embodiment of the invention is shown in Figures 7 to 9, and comprises a number of the same features as the patch 20 of the first embodiment, such features retaining the same reference numerals. A difference with the patch 20 of the second embodiment is that the patch 20 of the second embodiment does not include a rupturing element or support member disposed inside the fluid reservoir 23 defined by the housing 22 and membrane 21. The patch 20 of the second embodiment does include a rupturing element 27 and a support member 30 although these components are disposed outside the fluid reservoir 23.

The rupturing element 27 of the patch 20 of the second embodiment comprises a plate spaced from and substantially parallel to the membrane 21. The rupturing element 27 includes a plurality of pointed protrusions 29 extending from a surface of the plate proximate to the membrane 21 and extending in a direction towards the membrane 21. The support member 30 also comprises a plate, and is disposed between the rupturing element 27 and the membrane 21. The support element 30 includes a plurality of holes 40 located corresponding to the locations of the protrusions 29 on the adjacent rupturing element 27. The rupturing element 27 and the support member 30 respectively include a central hole 41, 42 aligned with the injection aperture 24 defined by the cylindrical wall 25 of the housing 22 and the hole 26 in the membrane 21.

The rupturing element 27 is moveably mounted to the support member 30. The rupturing element 27 is advantageously coupled to the support member 30 by one or more resilient coupling members 43. Such resilient coupling members 43 may comprise one or more springs or other suitable deflectable elements such as resilient arms or other portions of deflectable material bonded to the rupturing element 27 and to the support member 30, such as a foam or elastomeric block or other shaped element. Alternatively, the resilient coupling members 43 may be formed integrally with one of the rupturing element 27 and the support member 30 and bonded to the other of the rupturing element 27 and the support member 30. Yet further, the resilient coupling members 43 may be formed integrally with both the rupturing element 27 and the support member 30.

The rupturing element 27 is moveable between an initial position (shown in Figure 7) to an activated position (shown in Figure 8). The resilient coupling members 43 serve as a biasing arrangement 33 to bias the rupturing element 27 towards the initial position. In the initial position, the protrusions 29 are spaced from the membrane 21. The rupturing element 27 is moveable against a biasing force of the resilient coupling members 43 to the activated position, in which the protrusions 29 extend through the holes 40 in the support member 30 and into contact with the membrane 21 to pierce the membrane 21.

In the exemplary embodiment, a surface of the rupturing element 27 facing away from the membrane 21 comprises a skin-contacting surface of the patch 20. An adhesive 35 is provided on the outwardly-facing surface of the rupturing element 27 to enable the patch 20 to be adhered to the skin of a patient. A protective material layer 36 is provided on the outwardly facing surface of the rupturing element 27. The protective material layer 36 is removable from the rupturing element 27 before use of the patch 20, but serves to protect the skin-contacting surface of the rupturing element 27, and the adjacent membrane 21 from contact, contamination or damage prior to use, for example during manufacture, transport and storage of the patch 20.

As with the patch 20 of the first embodiment, the membrane 21 advantageously includes weakened regions 37 located facing the protrusions 29 of the rupturing element 27. The weakened regions 37 may be formed integrally with the membrane 21, and may comprise a region of reduced thickness of the material of the membrane 21, or a region demarcated by one or more lines of weakening in the material of the membrane 21, such as score lines or lines of reduced material thickness. Alternatively, the weakened region 37 may comprise an aperture in the membrane 21 and a portion of a different rupturable material bonded over the aperture.

Use of the patch 20 of the second embodiment will now be described, and is similar to the method of use of the patch 20 of the first embodiment, and so like method steps will not be repeated.

After a user has placed the patch 20 on the patient's skin and prepared the injector device 10 for administering an injection, the distal end of the injector device 10 is placed on the patch 20 with the axis A-A of the injector device 10 aligned with injection aperture 24. The user then presses the injector device 10 down onto the patch 20, which causes the rupturing element 27 to move from the initial position shown in Figure 7, to the activated position against the biasing force of the resilient coupling members 43. In the activated position, the pointed protrusions 29 of the rupturing element 27 extend through the holes 40 in the support member 30 and pierce the membrane 21. The piercing is facilitated by the protrusions 29 being located facing the weakened regions 37 of the membrane 21 such that those regions of the membrane are relatively easily pierced.

As with the patch 20 of the first embodiment, the pierced membrane allows the fluid from within the fluid reservoir 23 to be released onto the patient's skin at and around the injection site, with the associated advantageous effects described above. The patch 20 of the second embodiment may include one or more fluid passages 44 formed in the rupturing element 27 to enhance flow of a fluid from within the fluid reservoir 23 into contact with the skin S of the patient.

In a variant of the second embodiment intended within the scope of the present disclosure, fluid the reservoir may be disposed between the support plate 30 and the rupturing element 27. In such an alternative embodiment, relative movement of the rupturing element 27 towards the support plate 30 would similarly cause the piercing of the surface of the fluid reservoir 23 to release fluid therefrom. The support plate 30 and rupturing element 27 may be separate components connected together, or may be integrally formed components moveable relative to one another. The support plate 30 and rupturing element 27 may be connected by one or more resilient coupling members which act as a biasing arrangement biasing the support member 30 away from the rupturing element 27.

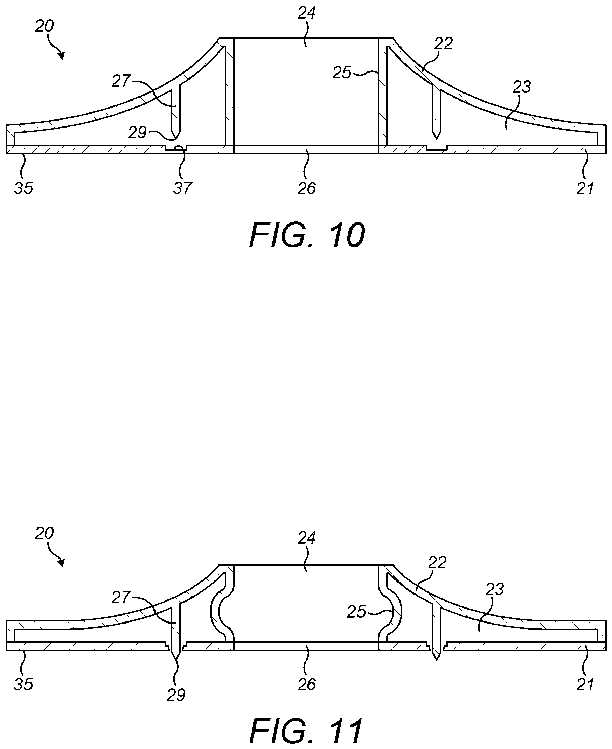

A patch 20 of a third embodiment of the invention is shown in Figures 10 and 11, and comprises a number of the same features as the patches 20 of the first and second embodiments, such features retaining the same reference numerals. The patch 20 of the third embodiment includes at least one rupturing element 27 disposed inside the fluid reservoir 23 which is defined by the housing 22 and membrane 21. The exemplary embodiment shown includes a plurality of rupturing elements 27. The rupturing elements 27 are provided extending from an interior surface of the housing 22 and are directed towards the membrane 21.

The rupturing elements 27 of the patch 20 of the third embodiment comprise protections 27 including pointed protrusions 29 at a tip of the protection 27 disposed proximate the membrane 21. The rupturing elements may be attached to the housing 22, or may be formed integrally with the housing 22, the latter being advantageous for ease and cost of manufacture.

The housing 22 is advantageously formed of a material to provide some structural strength to the patch 20, and some resistance to deformation of the housing 22 (as may also be the case with the patches 20 of the previously-described embodiments). Any suitable material may be chosen to provide the desired structural properties of the housing 22, such as metal or plastics, in an appropriate material thickness. The patch 20 is deformable between an initial position (shown in Figure 10) to an activated position (shown in Figure 11). As such, the rupturing elements 27 are moveable between an initial position to an activated position. The resilience of the housing 22 results in the rupturing elements 27 being biased into the initial position, and so as such, the patch 20 of the third embodiment includes a biasing arrangement 33 configured to bias the rupturing elements 27 into the initial position.

In the initial position, the protrusions 29 are spaced from the membrane 21. The rupturing elements 27 are moveable against a biasing force of the resilient housing 22 to the activated position, in which the protrusions 29 contact and pierce the membrane 21.

In the exemplary embodiment, a surface of the membrane 21 comprises a skin-contacting surface of the patch 20. An adhesive 35 is provided on the outwardly-facing surface of the membrane 21 to enable the patch 20 to be adhered to the skin of a patient. A protective material layer 36 is provided on the outwardly facing surface of the membrane 21. The protective material layer 36 is removable from the membrane 21 before use of the patch 20, but serves to protect the membrane from contact, contamination or damage prior to use, for example during manufacture, transport and storage of the patch 20.

As with the patches 20 of the first and second embodiments, the membrane 21 advantageously includes one or more weakened regions 37 located facing the protrusions 29 of the rupturing elements 27. The weakened regions 37 may be formed integrally with the membrane 21, and may comprise a region of reduced thickness of the material of the membrane 21, or a region demarcated by one or more lines of weakening in the material of the membrane 21, such as score lines or lines of reduced material thickness. Alternatively, the weakened regions 37 may comprise an aperture in the membrane 21 and a portion of a different rupturable material bonded over the aperture.

Use of the patch 20 of the third embodiment will now be described, and is similar to the method of use of the patch 20 of the first and second embodiments, and so like method steps will not be repeated.

After a user has placed the patch 20 on the patient's skin and prepared the injector device 10 for administering an injection, the distal end of the injector device 10 is placed on the patch 20 with the axis A-A of the injector device 10 aligned with injection aperture 24. The user then presses the injector device 10 down onto the patch 20, which causes the housing 22 to deform from the initial position to the activated position against the biasing force of the resilient deformable housing 22. Thereby, the rupturing elements 27 to move towards the membrane 21 such that the pointed protrusions 29 pierce the membrane 21. The piercing is facilitated by the protrusions 29 being located facing the weakened regions 37 of the membrane 21 such that those regions of the membrane are relatively easily pierced.

As with the patches 20 of the first and second embodiments, the pierced membrane 21 allows the fluid from within the fluid reservoir 23 to be released onto the patient's skin at and around the injection site, with the associated advantageous effects described above.

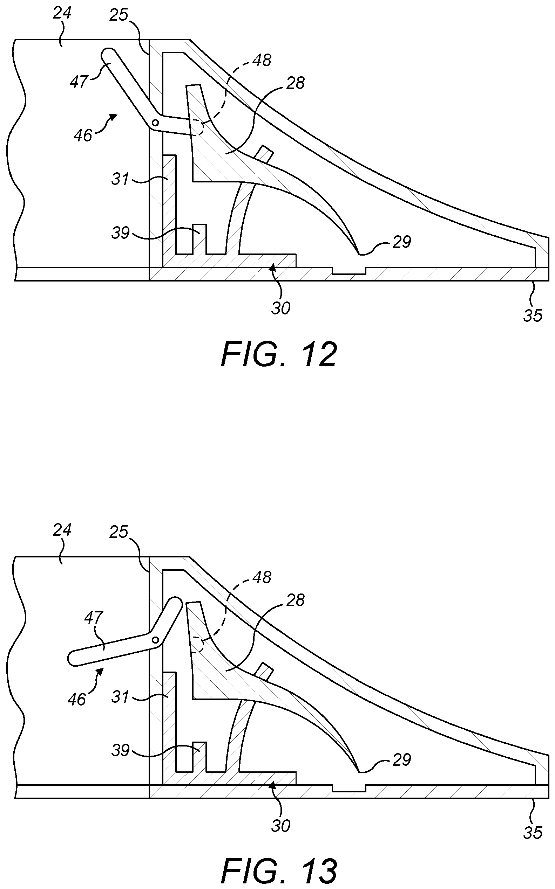

Figures 12 and 13 illustrate an enlarged view of a portion of a patch 20 of a fourth embodiment, and is similar to the patch of the first embodiment. A difference with the patch 20 of the fourth embodiment is that the patch 20 includes a locking mechanism 46. The locking mechanism 46 is associated with the rupturing element 27 and is configured such that in a locked condition, the rupturing element 27 is prevented from being moveable to rupture the fluid reservoir 23. In an unlocked condition, the rupturing element 27 is moveable to be able to rupture the fluid reservoir 23.

In the patch 20 of the exemplary fourth embodiment, the locking mechanism 46 is shown in a locked condition in Figure 12, and in an unlocked condition in Figure 13. The locking mechanism 46 comprises a pivotable locking arm 47 which in the locked condition, is engaged in a recess 48 in the rupturing element 27. The locking arm 47 is pivotably coupled to the cylindrical wall 25 of the housing 22, but alternatively may be pivotably coupled to the support member 30, for example to the hollow cylindrical portion 31 of the support member 30. In the locked condition, the locking arm 47 prevents the rupturing element 27 from moving relative to the housing 22 and the support member 30.

The locking arm 47 can be pivoted into the unlocked condition, in which the locking arm 47 is out of engagement with the recess 48 in the rupturing element 27. This renders the rupturing element 27 able to move relative to the housing 22 and the support member 30, and so the patch 20 to be used as described above.

The locking arm 47 may be manually moved from the locked condition to the unlocked condition by a user, for example by pressing the locking arm 47 with a finger. Alternatively, the locking arm 47 may be engaged by an injector device 10 being inserted into the injection aperture 24, and so the locking mechanism 46 rendered in the unlocked condition upon use of the patch 20 with an injector device 10. The locking mechanism 46 may therefore advantageously prevent accidental rupturing of the fluid reservoir 23 prior to use by a user, for example during manufacture, packaging, transport or storage.

The locking mechanism 46 shown in Figures 12 and 13 is one exemplary locking mechanism and alternative locking mechanisms are envisaged within the scope of the present disclosure. For example, other configurations of release elements other than the illustrated locking arm 47 may be provided. Such alternative release elements may comprise a sprung tab disengageable with the rupturing element, or a releasable pawl. Furthermore, and of the above described embodiments of patch 20 may include a locking mechanism, for example, with a release element comprising a breakable connection, such as a frangible pin extending between two components of the patch 20 which move relative to each other as the respective rupturing element 27 moves from the initial position to the activated position.
In the embodiments described, the membrane 21 comprises one or more weakened regions 37 which may advantageously facilitate rupturing of the fluid reservoir 23 by the rupturing element(s) 27. However, the membrane may optionally not include such weakened regions 37 within the scope of the present disclosure, and a membrane material and thickness may be appropriately selected such that the rupturing element(s) 27 may adequately pierce the membrane 21 without the presence of such weakened regions.

The protective material layer 36 advantageously may protect the membrane 21 or other skin-contacting surface of the patch 20 (such as the surface of the rupturing element in the second embodiment) from contact, contamination or damage prior to use of the patch 20, as described above. However, such protective layer 36 is not essential and may optionally be omitted in alternative embodiments.

The patch 20 may include an adhesive 35 on a skin-contacting surface, to facilitate the patch 20 locating in position on a patient's skin during an injection process. However, in an alternative embodiment, the adhesive 35 may be omitted.

The embodiments of patch 20 described above include a hole 26 in the membrane 21 through which a needle 17 of an injector device 10 may extend during use of the patch 20 in an injection process. However, alternative embodiments intended within the scope of the present disclosure may not include such hole 26, and the membrane 21 may comprise a continuous layer of material. Such an alternative may advantageously help ease of manufacture as alignment of the hole 26 with an injection aperture 24/cylindrical wall of a housing 25 would not be required. Furthermore, the membrane 21 may be easily pierced by the injection needle 17, thereby not presenting a problem to performing the injection process.

In the exemplary embodiments of patch 20 described above, the fluid in the fluid reservoir is described as possibly comprising a sterilising agent, antiseptic, analgesic or anaesthetic. However, it is intended that alternative fluids may be provided in the reservoir. In some embodiments, the function of the patch 20 may be to provide a distracting or comforting sensation to the patient's skin during the injection process. Such sensation may be provided by cooling or heating the skin. As such, the fluid may be any fluid capable of being heated or cooled such that upon contact with the patient's skin, the heat or cool sensation is detected by the patient as a distraction to the injection sensation. Such heating or cooling of the fluid may be achieved by heating or cooling the patch 20 before use. Such fluid may comprise any suitable fluid and may comprise an inert fluid, such as water for example. Such fluid may also comprise a fluid that endothermically or exothermically reacts with atmosphere once the reservoir is ruptured, to cool or heat the patient's skin S. Such fluid may also act to cool the patient's skin evaporatively once released from the reservoir onto the patient's skin, for example alcohol.

In embodiments in which the patch 20 includes a heating or cooling function to a patient's skin, the patch 20 may optionally include a temperature indicator, such as a thermochromic portion or other temperature indicating means. Such temperature indicator may change colour, or display a graphic or indicia when the temperature of the patch 20 or the patient's skin alters by a predetermined amount, or when a threshold temperature is achieved. This may, for example, be useful to indicate to a patient that the injection process can be initiated. Such an exemplary temperature indicator is shown as a thermochromic portion 45 in the patch 20 shown in Figure 2.

Although the biasing arrangement 33 of the first embodiment is illustrated in the form of resilient arms 33, alternative biasing arrangements may be provided, for example one or more springs or other suitable deflectable element(s) such one or more portions of deflectable material such as foam or elastomeric blocks or other shaped elements. In various embodiments of patch 20 described herein, comprising biasing arrangements 33 associated with the rupturing elements 27, the rupturing elements 27 may advantageously be biased to return to the initial position upon release of a force which moved the respective rupturing element from the initial position to the activated position.

In the first embodiment of patch 20, a stop arrangement 38 is provided, by the stop posts 39. However, alternative configurations of stop arrangements are envisaged, which may comprise a biasing means or arms being in a fully deflected state (such as the resilient arms 33 being at a maximum deflected state). In the patch 20 of the second embodiment, the stop arrangement may be provided by the resilient coupling members 43 being compressed to a maximum extent, or alternatively when the plate of the rupturing element 27 being in contact with the plate of the support member 30.

The patch 20 of the second embodiment illustrated and described above, includes one arrangement of rupturing mechanism for rupturing the fluid reservoir 23. However, alternative configurations of external rupturing elements are intended within the scope of the present disclosure.

The exemplary embodiments of patch 20 described and illustrated herein include an injection aperture 24 through which an injection needle 17 extends during use of the patch 20 in an injection process. This may at least partially conceal the injection needle 17 from view of the patient during an injection process. This may be advantageous for patients who are anxious about injections and needles and thereby may help relax the patient during an injection process. In some embodiments, the material of construction of the patch 20, such as the second layer/housing 22, may comprise an opaque material to further help conceal the injection needle 17 from view of a patient. Alternatively, however, the material of construction of the patch 20, such as the second layer/housing 22, may comprise a translucent or semi-translucent material to enable fluid within the fluid reservoir to be visible by the patient. This may help to make operation of the patch 20, including rupturing of the reservoir and release of the fluid therein, be visible to a patient. This may provide reassurance of operation to the patient.

The embodiments of patch 20, with the exemplary injection apertures 24, may also advantageously help locate an injector device 10 at the injection site during use by a patient. This can help toward preventing incorrect use or alignment of the injector device 10, or damage or injury by the injector device during use.

The exemplary embodiments of patch 20 described above, comprise a fluid reservoir 23 defined by the first and second layers 21, 22 of the patch 20. However, in alternative embodiments intended within the scope of the present disclosure, a fluid reservoir 23 may be provided between the first and second layers, but within a further layer or layers of material, for example within a balloon or pocket of fluid disposed between the first and second layers of the patch 20. As such, a rupturing element may be disposed between the first and second layers of the patch, but outside of the fluid reservoir.

The exemplary embodiments of patch 20 described above, comprise an injection aperture 24. However, in alternative embodiments intended within the scope of the present disclosure, the injection aperture may be omitted. In use of such an alternative configuration of patch 20, the needle 17 of an injector device 10 may pierce through the body of the patch 20 before piercing the patient's skin S at the injection site.

As used herein, the term "rupture" is intended to mean break, burst, tear, puncture or otherwise jeopardise the integrity of the sealed state of the sealed fluid reservoir to permit relese of the fluid contained therein from the reservoir.

The various exemplary embodiments of patch 20 described herein, with various optional features mentioned above, may advantageously serve to provide one or more patient benefits of injection pain reduction, distraction from injection needle sensation, even of only by use of the patch 20 per se, needle anxiety reduction, and generally provide improved patient usability and acceptance of use of injector devices.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injector device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastrointestinal tract. The presently described drugs may be particularly useful with injector devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyhepta¬decanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

## Claims

1. A patch (20) for placement on the skin and for use with an injector device (10), the patch comprising:
a first layer (21) on a first side of the patch;
a second layer (22) on a second side of the patch opposite to the first side;
a sealed fluid reservoir (23) between the first and second layers;
a fluid contained within the fluid reservoir;
a moveable rupturing element (27) configured to pierce and rupture the fluid reservoir to allow release of the fluid out of the fluid reservoir; and
an aperture (24) extending at least partially through the patch to receive an injection needle (17) of an injector device.

2. A patch (20) according to claim 1 wherein the fluid reservoir (23) is defined by the first and second layers (21, 22).

3. A patch (20) according to any preceding claim wherein the rupturing element (27) is moveable between an initial position and an activated position in which the rupturing element ruptures the fluid reservoir (23).

4. A patch (20) according to claim 3 further comprising a biasing arrangement (33) configured to bias the rupturing element (27) into the initial position.

5. A patch (20) according to any preceding claim, wherein the rupturing element (27) is disposed within the fluid reservoir (23).

6. A patch (20) according to any preceding claim, wherein the patch comprises a support member (30).

7. A patch (20) according to claim 6 wherein the rupturing element (27) is moveably mounted to the support member (30).

8. A patch (20) according to any preceding claim, wherein the rupturing element (27) is integrally formed with the one of the first and second layers (21, 22).

9. A patch (20) according to any of claims 1 to 4 or 6 to 8, wherein the rupturing element (27) is disposed outside the fluid reservoir (23).

10. A patch (20) according to any preceding claim, wherein the injection aperture (24) extends through the first and second layers (21, 22) of the patch.

11. A patch (20) according to any preceding claim, wherein the first layer (21) comprises a piercable membrane which can be pierced by the rupturing element (27) to rupture the fluid reservoir (23), and optionally wherein the piercable membrane includes one or more regions of weakness at which the piercable membrane is more easily ruptured than at other regions of the piercable membrane, and optionally wherein the at least one region of weakness comprises a region of reduced thickness in the piercable membrane.

12. A patch (20) according to any preceding claim, wherein the patch includes a locking mechanism (46) associated with the rupturing element (27) and configured such that in a locked condition of the locking mechanism, the rupturing element is prevented from being moveable to rupture the fluid reservoir (23), and in an unlocked condition of the locking mechanism, to rupturing element is moveable to be able to rupture the fluid reservoir.

13. A patch (20) according to any preceding claim, wherein the patch includes a stop (38) associated with the rupturing element (27) and configured to restrict movement of the rupturing element beyond a predetermined range of movement.

14. A system comprising a patch (20) according to any of claims 1 to 13, and an injector device (10) for use with the patch, wherein the injector device comprises a housing (22) configured to receive a container of medicament, and wherein the injector device optionally comprises a container of medicament received within the housing (22).

15. A method of use of a patch (20) according to any of claims 1 to 13, the method comprising moving the moveable rupturing element to pierce and rupture the fluid reservoir to allow release of the fluid out of the fluid reservoir.

## Patentansprüche

1. Patch (20) zur Platzierung auf der Haut und zur Verwendung mit einer Injektorvorrichtung (10), wobei das Patch Folgendes umfasst:
eine erste Schicht (21) auf einer ersten Seite des Patchs,
eine zweite Schicht (22) auf einer der ersten Seite gegenüberliegenden zweiten Seite des Patchs,
ein abgedichtetes Fluidreservoir (23) zwischen der ersten und der zweiten Schicht,
ein in dem Fluidreservoir enthaltenes Fluid,
ein bewegliches Berstelement (27), das dazu ausgestaltet ist, das Fluidreservoir zu durchstechen und zu bersten, um eine Freisetzung des Fluids aus dem Fluidreservoir zu gestatten, und
eine Öffnung (24), die sich zur Aufnahme einer Injektionsnadel (17) einer Injektorvorrichtung zumindest teilweise durch das Patch erstreckt.

2. Patch (20) nach Anspruch 1, wobei das Fluidreservoir (23) durch die erste und die zweite Schicht (21, 22) definiert ist.

3. Patch (20) nach einem der vorhergehenden Ansprüche, wobei das Berstelement (27) zwischen einer Ausgangsposition und einer aktivierten Position, in der das Berstelement das Fluidreservoir (23) zerbirst, beweglich ist.

4. Patch (20) nach Anspruch 3, ferner umfassend eine Vorspannungsanordnung (33), die zum Vorspannen des Berstelements (27) in die Ausgangsposition ausgestaltet ist.

5. Patch (20) nach einem der vorhergehenden Ansprüche, wobei das Berstelement (27) in dem Fluidreservoir (23) angeordnet ist.

6. Patch (20) nach einem der vorhergehenden Ansprüche, wobei das Patch ein Stützglied (30) umfasst.

7. Patch (20) nach Anspruch 6, wobei das Berstelement (27) beweglich an dem Stützglied (30) montiert ist.

8. Patch (20) nach einem der vorhergehenden Ansprüche, wobei das Berstelement (27) integral mit der einen der ersten und der zweiten Schicht (21, 22) ausgebildet ist.

9. Patch (20) nach einem der Ansprüche 1 bis 4 oder 6 bis 8, wobei das Berstelement (27) außerhalb des Fluidreservoirs (23) angeordnet ist.

10. Patch (20) nach einem der vorhergehenden Ansprüche, wobei sich die Injektionsöffnung (24) durch die erste und die zweite Schicht (21, 22) des Patchs erstreckt.

11. Patch (20) nach einem der vorhergehenden Ansprüche, wobei die erste Schicht (21) eine durchstechbare Membran umfasst, die von dem Berstelement (27) durchstochen werden kann, um das Fluidreservoir (23) zu bersten, und optional wobei die durchstechbare Membran einen oder mehrere Schwächungsbereiche aufweist, in denen die durchstechbare Membran leichter zerbirst als in anderen Bereichen der durchstechbaren Membran, und optional wobei der mindestens eine Schwächungsbereich einen Bereich reduzierter Dicke in der durchstechbaren Membran umfasst.

12. Patch (20) nach einem der vorhergehenden Ansprüche, wobei das Patch einen dem Berstelement (27) zugeordneten Verriegelungsmechanismus (46) aufweist, der so ausgestaltet ist, dass in einem verriegelten Zustand des Verriegelungsmechanismus verhindert wird, dass das Berstelement zum Bersten des Fluidreservoirs (23) beweglich ist, und in einem entriegelten Zustand des Verriegelungsmechanismus das Berstelement beweglich ist, um das Fluidreservoir bersten zu können.

13. Patch (20) nach einem der vorhergehenden Ansprüche, wobei das Patch einen dem Berstelement (27) zugeordneten Anschlag (38) aufweist, der dazu ausgestaltet ist, eine Bewegung des Berstelements über einen vorbestimmten Bewegungsbereich hinaus zu beschränken.

14. System, umfassend ein Patch (20) nach einem der Ansprüche 1 bis 13 und eine Injektorvorrichtung (10) zur Verwendung mit dem Patch, wobei die Injektorvorrichtung ein Gehäuse (22) umfasst, das zur Aufnahme eines Medikamentenbehälters ausgestaltet ist, und wobei die Injektorvorrichtung optional einen in dem Gehäuse (22) aufgenommenen Medikamentenbehälter umfasst.

15. Verfahren zur Verwendung eines Patchs (20) nach einem der Ansprüche 1 bis 13, wobei das Verfahren das Bewegen des beweglichen
Berstelements zum Durchstechen und Bersten des Fluidreservoirs, um eine Freisetzung des Fluids aus dem Fluidreservoir zu gestatten, umfasst.

## Revendications

1. Timbre (20) destiné à être placé sur la peau et à être utilisé avec un dispositif d'injection (10), le timbre comprenant :
une première couche (21) sur un premier côté du timbre ;
une seconde couche (22) sur un second côté du timbre opposé au premier côté ;
un réservoir de fluide scellé (23) entre les première et seconde couches ;
un fluide contenu dans le réservoir de fluide ;
un élément de rupture mobile (27) configuré pour percer et rompre le réservoir de fluide afin de permettre la libération du fluide hors du réservoir de fluide ; et
une ouverture (24) s'étendant au moins partiellement à travers le timbre pour recevoir une aiguille d'injection (17) d'un dispositif d'injection.

2. Timbre (20) selon la revendication 1 dans lequel le réservoir de fluide (23) est défini par les première et seconde couches (21, 22).

3. Timbre (20) selon une quelconque revendication précédente dans lequel l'élément de rupture (27) est mobile entre une position initiale et une position activée dans laquelle l'élément de rupture rompt le réservoir de fluide (23).

4. Timbre (20) selon la revendication 3 comprenant en outre un agencement de sollicitation (33) configuré pour solliciter l'élément de rupture (27) dans la position initiale.

5. Timbre (20) selon une quelconque revendication précédente, dans lequel l'élément de rupture (27) est disposé à l'intérieur du réservoir de fluide (23).

6. Timbre (20) selon une quelconque revendication précédente, dans lequel le timbre comprend un élément de support (30).

7. Timbre (20) selon la revendication 6 dans lequel l'élément de rupture (27) est monté de manière mobile sur l'élément de support (30).

8. Timbre (20) selon une quelconque revendication précédente, dans lequel l'élément de rupture (27) est formé d'un seul tenant avec l'une des première et seconde couches (21, 22).

9. Timbre (20) selon l'une quelconque des revendications 1 à 4 ou 6 à 8, dans lequel l'élément de rupture (27) est disposé à l'extérieur du réservoir de fluide (23).

10. Timbre (20) selon une quelconque revendication précédente, dans lequel l'ouverture d'injection (24) s'étend à travers les première et seconde couches (21, 22) du timbre.

11. Timbre (20) selon une quelconque revendication précédente, dans lequel la première couche (21) comprend une membrane perçable qui peut être percée par l'élément de rupture (27) pour rompre le réservoir de fluide (23), et facultativement dans lequel la membrane perçable comporte une ou plusieurs régions de faiblesse au niveau desquelles la membrane perçable est plus facilement rompue qu'au niveau d'autres régions de la membrane perçable, et éventuellement dans lequel l'au moins une région de faiblesse comprend une zone d'épaisseur réduite dans la membrane perçable.

12. Timbre (20) selon une quelconque revendication précédente, dans lequel le timbre comporte un mécanisme de verrouillage (46) associé à l'élément de rupture (27) et configuré de telle sorte que dans un état verrouillé du mécanisme de verrouillage, l'élément de rupture soit empêché d'être mobile pour rompre le réservoir de fluide (23), et que dans un état déverrouillé du mécanisme de verrouillage, l'élément de rupture soit mobile pour pouvoir rompre le réservoir de fluide.

13. Timbre (20) selon une quelconque revendication précédente, dans lequel le timbre comporte une butée (38) associée à l'élément de rupture (27) et configurée pour restreindre le mouvement de l'élément de rupture au-delà d'une plage prédéfinie de mouvement.

14. Système comprenant un timbre (20) selon l'une quelconque des revendications 1 à 13, et un dispositif d'injection (10) destiné à être utilisé avec le timbre, dans lequel le dispositif d'injection comprend un logement (22) configuré pour recevoir un récipient de médicament, et dans lequel le dispositif d'injection comprend facultativement un récipient de médicament reçu à l'intérieur du logement (22).

15. Procédé d'utilisation d'un timbre (20) selon l'une quelconque des revendications 1 à 13, le procédé comprenant le déplacement de
l'élément de rupture mobile pour percer et rompre le réservoir de fluide afin de permettre la libération du fluide hors du réservoir de fluide.
